# EUROPÄISCHE PATENTSCHRIFT

(11) **EP 0 673 948 B1**
(45) Veröffentlichungstag und Bekanntmachung des Hinweises auf die Patenterteilung: **11.11.1998**
(21) Anmeldenummer: 95101991.8
(22) Anmeldetag: 14.02.1995
(51) Int. Cl.: C07K 14/16, G01N 33/569, C07K 16/10

(54) **Von einem Retrovirus aus der HIV-Gruppe abgeleitete Peptide und deren Verwendung**
Pepticles derived from a retrovirus of the HIV-group and their use
Peptides dérivés d'un rétrovirus du groupe SIV et leur utilisation

(30) Priorität: 23.02.1994 DE 4405810
(43) Veröffentlichungstag der Anmeldung: 27.09.1995
(73) Patentinhaber: Dade Behring Marburg GmbH, 35001 Marburg (DE)
(72) Erfinder: Brust, Stefan, Dr., D-35041 Marburg-Michelbach (DE); Knapp, Stefan, Dr., D-35041 Marburg (DE); Gerken, Manfred, Dr., D-35041 Marburg (DE); Gürtler, Lutz G., Prof. Dr., D-81249 München (DE)
(74) Vertreter: Keller, Günter, Dr.

(56) Entgegenhaltungen:
- EP-A- 0 345 375
- EP-A- 0 591 914
- THE LANCET, Bd. 340, 1992 Seiten 681-82, II.AGUT ET AL. 'Isolation of atypical HIV-1-related retrovirus from AIDS patient'

## Beschreibung

Die vorliegende Erfindung betrifft synthetische Peptide und rekombinante Proteine, die von einem neuen Retrovirus aus der HIV-Gruppe, MVP5180/91, abgeleitet wurden. Beschrieben werden Verfahren zur Herstellung solcher Peptide und rekombinanter Proteine. Die Erfindung betrifft weiterhin den Einsatz dieser Peptide und rekombinanten Proteine für medizinische Zwecke, insbesondere für die Diagnostik und für die Herstellung von Impfstoffen.

Retroviren, die zur sogenannten HIV-Gruppe gehören, führen bei damit infizierten Menschen zu Krankheitserscheinungen, die unter dem Sammelbegriff Immunschwäche bzw. AIDS (Acquired Immune Deficiency Syndrome) zusammengefaßt werden.

Epidemiologische Studien belegen, daß das Humane Immunschwäche Virus (HIV) das aetiologische Agens für die überwiegende Mehrheit der AIDS (Acquired Immune Deficiency Syndrome)-Fälle darstellt. Ein 1983 aus einem Patienten isoliertes und charakterisiertes Retrovirus erhielt die Bezeichnung HIV-1 (Barré-Sinoussi, F. et al., Science **220**, 868-871 [1983]). Eine Variante von HIV-1 wird in WO 86/02383 beschrieben.

Bis 1993 wurden die bekannten HIV 1-Isolate aufgrund von Sequenzvergleichen und epidemiologischen Gesichtspunkten in die fünf Subtypen A - E eingeordnet (G.Myers et al., 1992, Human Retroviruses and AIDS 1992. A compilation and analysis of nucleic acid and aminoacid sequences. Los Alamos Laboratory, Los Alamos, USA).

Eine zweite Gruppe von Humanen Immunschwäche Viren wurde 1985 in Westafrika identifiziert (Clavel, F. et al., Science **233**, 343-346 [1986]) und als Humanes Immunschwäche Virus Typ 2 (HIV-2) bezeichnet (EP-A-0 239 425). HIV-2 Retroviren unterscheiden sich deutlich von HIV-1, weisen jedoch auch eine Verwandtschaft zu Affen-Immunschwäche Viren SIV auf. Wie HIV-1 führt auch HIV-2 zu einer AIDS-Symptomatik.

Eine weitere Variante eines Immunschwäche Retrovirus wird in der EP-A-0 345 375 beschrieben und dort als HIV-3 Retrovirus bezeichnet (ANT 70).

Auch in Lancet Vol. 340, Sept. 1992, s. 681-682 wird die Isolierung eines weiteren, varianten Immunschwächevirus beschrieben.

Es ist ein Charakteristikum der Humanen Immunschwäche Viren, daß sie eine hohe Variabilität aufweisen, die die Vergleichbarkeit der verschiedenen Isolate deutlich kompliziert. Beim Vergleich diverser HIV-1-Isolate treten z.B. in einigen Regionen des Genoms hohe Variabilitäten auf, während andere Genombereiche vergleichsweise konserviert vorliegen (Benn, S. et al. Science **230**, 949-951 [1985]). Ein wesentlich größerer Polymorphismus konnte auch für HIV-2 beobachtet werden (Clavel, F. et al., Nature **324**, 691-695 [1986]). Die größte genetische Stabilität besitzen Bereiche in den gag und pol Genen, die für strukturell und enzymatisch essentielle Proteine codieren; einige Regionen im env-Gen sowie die Gene (vif, vpr, tat, rev, nef), die für regulatorische Proteine codieren, zeigen einen hohen Grad an Variabilität. Es konnte weiterhin gezeigt werden, daß Antisera gegen HIV-1 auch mit gag und pol Genprodukten von HIV-2 kreuzreagieren, obwohl nur geringe Sequenzhomologie vorlag. Ebenfalls war die Hybridisierung zwischen diesen beiden Viren wenig signifikant, wenn nicht sehr wenig stringente Konditionen verwandt wurden (Clavel, F. et al., Nature **324**, 691-695 [1986]).

Aufgrund der weiten Verbreitung der Retroviren aus der HIV-Gruppe und der Tatsache, daß zwischen dem Zeitpunkt der Infektion und dem Zeitpunkt, zu dem eindeutige Symptome für pathologische Veränderungen erkennbar sind, ein Zeitraum von einigen bis vielen Jahren (2-20) liegt, ist es epidemiologisch von großer Bedeutung, die Infektion mit Retroviren der HIV-Gruppe möglichst frühzeitig und vor allem zuverlässig zu bestimmen. Dies spielt nicht nur eine Rolle bei der Diagnose von Patienten, die Zeichen von Immunschwäche aufweisen, sondern auch bei der Überprüfung von Blutspendern. Es hat sich herausgestellt, daß bei der Verwendung von Retroviren oder Bestandteilen davon des Types HIV-1 oder HIV-2 in Nachweissystemen bei manchen Seren kein oder nur ein schwacher Nachweis von Antikörpern geführt werden kann, obwohl bei den Patienten, von denen die Seren stammen, Zeichen von Immunschwäche auftreten. Mit Hilfe der erfindungsgemäßen Antigene ist in bestimmten Fällen ein derartiger Nachweis möglich.
In der DE 43 18 186 sowie der korrespondierenden, nicht vorveröffentlichten europäischen Patentanmeldung 93.116058.4 wird die Isolation und Charakterisierung eines neuen, als MVP5180/91 bezeichneten Immunschwäche-Virus beschrieben, das 1991 aus peripheren Lymphozyten einer 34jährigen Patientin aus Kamerun isoliert wurde, die Zeichen von Immunschwäche aufwies. Geographisch stammt dieses Retrovirus aus einer Region in Afrika, die zwischen Westafrika mit endemischer HIV 1- und HIV 2-Virusinfektion und Ostafrika mit fast ausschließlicher HIV 1-Verbreitung lokalisiert ist. Außerdem werden in der Patentanmeldung Nukleotid-Sequenzen des Virusgenoms von MVP5180/91 und davon abgeleitete Aminosäuresequenzen beschrieben. Dieses Retrovirus wurde bei der European Collection of Animal Cell Cultures (ECACC) unter der Nummer V 920 92 318 gemäß den Bedingungen des Budapester Vertrages hinterlegt.

Ebenso wie HIV-1 und HIV-2 wächst das erfindungsgemäße MVP-5180/91 in folgenden Zellinien: HUT 78, Jurkat-Zellen, C8166-Zellen und MT-2-Zellen. Die Isolierung und Vermehrung von Viren wird in dem Buch "Viral Quantitation in HIV Infection, Editor Jean-Marie Andrieu, John Libbey Eurotext, 1991" ausführlich beschrieben. Die dort beschriebenen Arbeitsmethoden werden durch Bezugnahme zum Gegenstand der Offenbarung der vorliegenden Anmeldung gemacht.

Weiterhin besitzt das erfindungsgemäße Virus eine magnesiumabhängige Reverse Transkriptase, die aber nicht manganabhängig ist. Dies stellt eine weitere Gemeinsamkeit zu den Viren HIV-1 und HIV-2 dar.

Zum besseren Verständnis der Unterschiede des erfindungsgemäßen MVP5180/91-Virus zu den Retroviren HIV 1 und HIV 2 soll zunächst kurz der Aufbau der Immunschwäche verursachenden Retroviren erläutert werden. Im Inneren des Virus befindet sich die RNA in einem kegelförmigen Core, das aus Proteinuntereinheiten zusammengesetzt ist, die die Bezeichnung p24 (p für Protein) tragen. Dieses innere Core wird von einer Proteinhülle umgeben, die aus dem Protein p17 aufgebaut ist (äußeres Core). Das Virus wird von einer Glykoproteinhülle umgeben, die neben Lipiden und anderen Bestandteilen das Transmembranprotein gp41 und das äußere Membranprotein gp120 enthält. Dieses gp120 kann mit den CD-4-Rezeptoren der Wirtszellen eine Bindung eingehen.

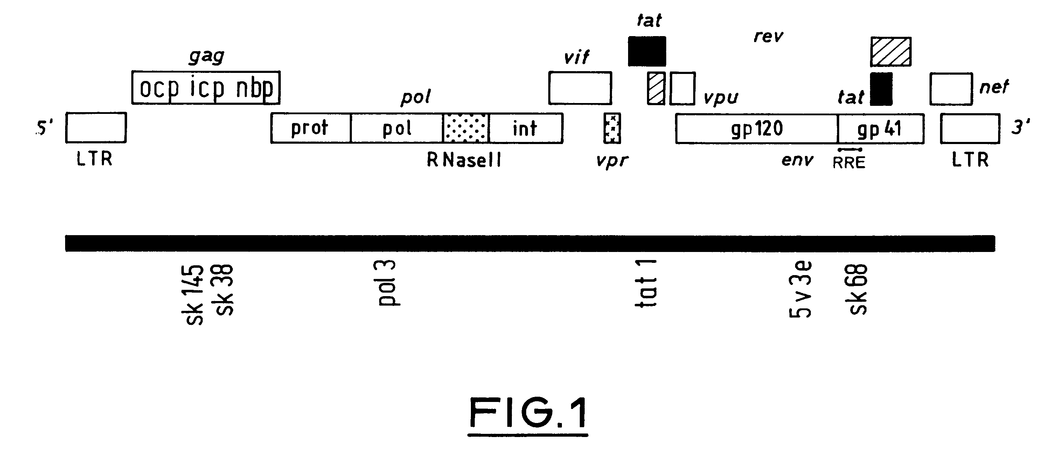

Soweit bekannt, weist die RNA der HIV-Viren - vereinfacht dargestellt - folgende Genbereiche auf: An den beiden Enden sogenannte long terminal repeats (LTR), und die folgenden Genbereiche gag, pol, env und nef. Das Gen gag codiert unter anderem für die Kern(Core)-Proteine, p 24 und p 17, das Gen pol codiert u.a. für die Reverse Transkriptase, die RNAse H und die Integrase und das Gen env codiert für die Glykoproteine gp 41 und gp 120 der Virushülle. Das Gen nef codiert für ein Protein mit Regulatorfunktion. Eine schematische Anordnung des Genomes von Retroviren des HIV-Typs ist in Figur 1 gezeigt.

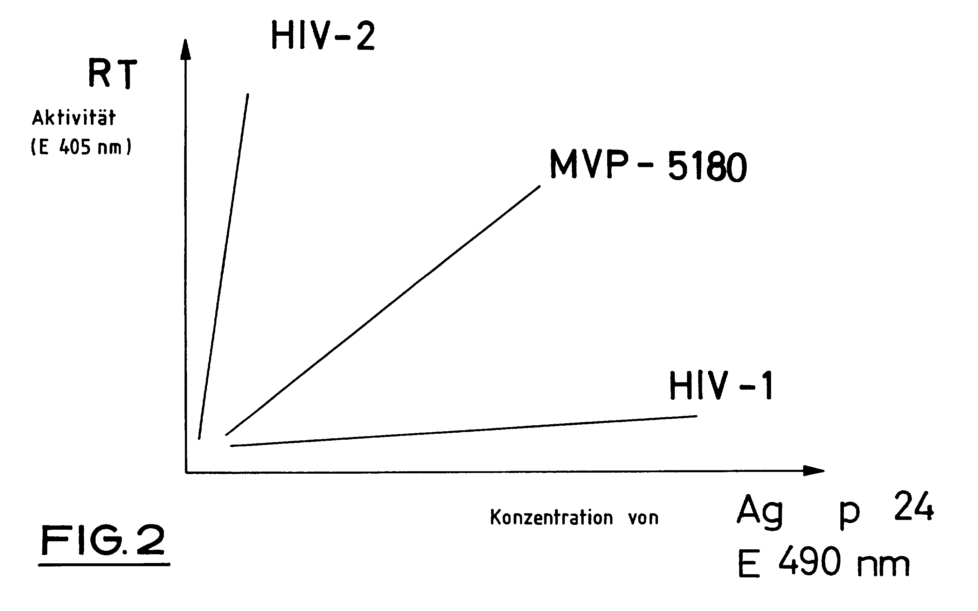

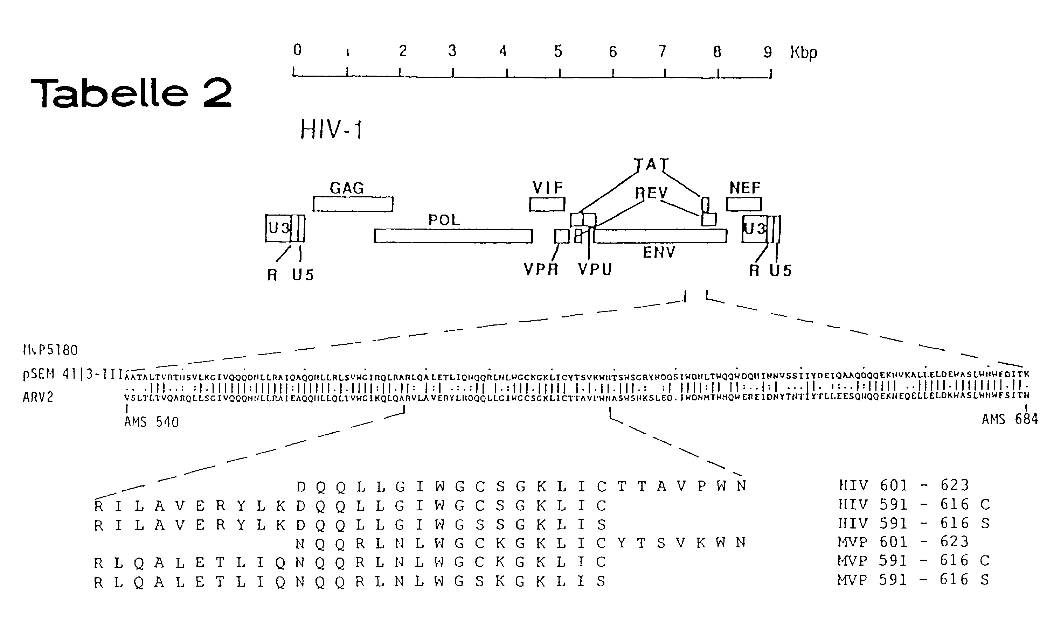

Eine Unterscheidung zwischen den Retroviren HIV-1 und HIV-2 ist u.a. dadurch möglich, daß virales Antigen ausgetestet wird mit einem monoklonalen Antikörper, der kommerziell als Testkit von Abbott (HIVAG-1 Monoclonal) erhältlich ist, und gegen das (HIV-1) p 24 gerichtet ist. Es ist bekannt, daß der Gehalt an Reverser Transkriptase in den Virustypen HIV-1 und HIV-2 etwa gleich ist. Wenn man deshalb in Verdünnungen der aufgeschlossenen Viren die Extinktion (E 490 nm), erhalten durch die Antigen-Antikörper-Reaktion, aufträgt gegen die Aktivität der Reversen Transkriptase, dann erhält man eine Graphik, die etwa der Figur 2 entspricht. Hierbei stellt man fest, daß im Verhältnis zu dem Gehalt an Reverser Transkriptase bei HIV-1 eine sehr hohe Bindungsaffinität für p 24 mit dem eingesetzten monoklonalen Antikörper vorhanden ist. Für HIV-2 tritt dagegen nur eine sehr geringe Bindungsaffinität für p 24 bei Einsatz des monoklonalen Antikörpers wiederum bezogen auf den Gehalt an Reverser Transkriptase auf. Werden diese Messungen durchgeführt für MVP-5180/91, dann befindet sich die Kurve ziemlich genau in der Mitte zwischen der Kurve von HIV-1 und HIV-2, d.h. die Bindungsaffinität des monoklonalen Antikörpers gegen MVP-5180/91 p 24 ist gegenüber HIV-1 reduziert. Figur 2 zeigt schematisch diesen Sachverhalt, wobei RT Reverse Transkriptase bedeutet und als Antigen (Ag) das Protein p 24 eingesetzt wird, gegen das der monoklonale Antikörper, der in dem von Abbott käuflich erwerblichen Testkit vorhanden ist, gerichtet ist.

Ein sehr vielseitig verwendbares System der Gentechnologie ist die sogenannte PCR (polymerase chain reaction) geworden, wobei die zur Durchführung des Verfahrens benötigten Komponenten käuflich erworben werden können. Mit diesem Verfahren ist es möglich, DNA-Sequenzen zu amplifizieren, wenn DNA-Bereiche der zu amplifizierenden Sequenz bekannt sind. Es müssen dann kurze komplementäre DNA-Fragmente (Oligonucleotide = Primer) synthetisiert werden, die sich an einen kurzen Bereich der zu amplifizierenden Nukleinsäuresequenz anlagern. Für die Testdurchführung werden HIV-Nukleinsäuren mit den Primern zusammengebracht in einer Reaktionsmischung, die zusätzlich eine Polymerase und Nukleotidtriphosphate enthält. Die Polymerisation (DNA-Synthese) wird für eine bestimmte Zeit durchgeführt und dann werden die Nukleinsäurestränge durch Erwärmen getrennt. Nach Abkühlen läuft dann die Polymerisation erneut an. Wenn es sich also bei dem erfindungsgemäßen Retrovirus um ein HIV-1 oder HIV-2 Virus handelt, dann müßte die Nukleinsäure amplifiziert werden können, indem Primer verwendet werden, die konserviert sind innerhalb der bekannten Sequenzen der Viren HIV-1 und HIV-2. Derartige Primer sind zum Teil vorbeschrieben (Lauré, F. et al., Lancet ii, (1988) 538-541 für pol 3 und pol 4 bzw. Ou C.Y. et al., Science 239 (1988) 295-297 für sk 38/39, sk 68/69).

Es wurde nun herausgefunden, daß bei Verwendung der vorbeschriebenen Primerpaare keine oder nur schwache Amplifikate der MVP5180/91 DNA erhalten wurden (DE 43 18 186).

Eine weitverbreitete Methode zum Nachweis von HIV-Antikörpern ist der sogenannte Western Blot (Immunoblot). Dabei werden die viralen Proteine gelelektrophoretisch aufgetrennt und dann auf eine Membran überführt. Die mit den überführten Proteinen versehenen Membranen werden dann mit Seren der zu untersuchenden Patienten in Verbindung gebracht. Sofern Antikörper gegen die viralen Proteine vorhanden sind, binden diese an die Proteine. Nach Waschen verbleiben lediglich spezifische Antikörper gegen virale Proteine. Die Antikörper werden dann mit Antiantikörpern sichtbar gemacht, die regelmäßig mit einem Enzym gekoppelt sind, das eine Farbreaktion katalysiert. Auf diese Weise können die Banden der viralen Proteine sichtbar gemacht werden.

Das HIV-Isolat MVP5180/91 weist gegenüber den Viren HIV 1 und HIV 2 im Western Blot zwei signifikante wesentliche Unterschiede auf. Das HIV-1 zeigt regelmäßig eine starke Bande, die dem Protein p24 zuzuordnen ist und eine sehr schwache, oft kaum sichtbare Bande, die dem Protein p23 zuzuordnen ist. HIV-2 weist eine kräftige Bande auf, die dem Protein p25 zuzuordnen ist und manchmal eine schwache Bande, die dem Protein p23 zuzuordnen ist. Im Unterschied dazu weist MVP5180/91 Virus zwei etwa gleich starke Banden auf, die den Proteinen p24 und p25 entsprechen.

Ein weiterer signifikanter Unterschied besteht bei den Banden, die der Reversen Transkriptase zuzuordnen sind. HIV 1 zeigt eine Bande (p53), die der Reversen Transkriptase entspricht und eine Bande (p66), die der Reversen Transkriptase verbunden mit der RNAse H entspricht. Bei HIV 2 entspricht die Reverse Transkriptase dem Protein p55 und, wenn sie mit der RNAse H verbunden ist, dem Protein p68. Das HIV-Isolat MPV5180/91 weist dagegen eine Bande auf bei dem Protein p48, die der Reversen Transkriptase entspricht, und eine Bande, bei dem Protein p60, die der Reversen Transkriptase in Verbindung mit RNAse H entspricht.

Aus diesen Daten kann geschlossen werden, daß die Reverse Transkriptase des MVP5180/91 ein Molekulargewicht hat, das zwischen etwa 3 und etwa 7 Kilodalton kleiner ist als das der Reversen Transkriptase von HIV 1 bzw. HIV 2.

Es wurde herausgefunden, daß mit Hilfe des Virus MVP5180/91 anti-env-Antikörper in Seren von deutschen Patienten, die Zeichen von Immunschwäche zeigen, nur schwach nachgewiesen werden können, wobei die Seren aber stark reagieren, wenn anstelle von MVP5180/91 ein HIV 1-Virus verwendet wird (DE 43 18 186). Diese stärkere Nachweisreaktion wurde vor allem lokalisiert in dem gp41 Protein. Bei den Versuchen wurden Serumpanels gegenübergestellt, die einmal von deutschen Patienten stammen und zum anderen von afrikanischen Patienten mit Zeichen von Immunschwäche.

Die Klonierung und Sequenzierung des Genoms von MVP5180/91 wurde bereits beschrieben (DE 43 18 186).

Zwischen verschiedenen Virusisolaten kann die Ähnlichkeit durch den Grad der Homologie der Nukleinsäure- oder Proteinsequenzen ausgedrückt werden. Eine 50 %ige Homologie bedeutet beispielsweise, daß 50 von 100 Nukleotid- oder Aminosäure-Positionen in den Sequenzen übereinstimmen.

Die Homologie von Proteinen wird durch Sequenzanalyse bestimmt. Homologe DNA-Sequenzen lassen sich auch durch die Hybridisierungstechnik nach Southern (Southern E. M., J. Mol. Biol. 98: 503-517, 1975) ermitteln.

Eine Zusammenfassung des Sequenzvergleichs zwischen MVP5180/91 und den Konsensus-Sequenzen von HIV 1 und HIV 2 sowie dem Isolat ANT70, einem als HIV 3 bezeichneten Virus (WO 89/12094 bzw. EP-A-0 345 375) ist in Tabelle 1 gezeigt. Dieser Sequenzvergleich macht deutlich, daß MVP 5180/91 z.B. in dem diagnostisch wichtigen env-Genbereich zu HIV 1 nur 53 % und zu HIV 2 nur 49 % homologe Sequenzen besitzt. Im Unterschied dazu zeigen z.B. HIV 1-Isolate der Subtypen A-E unter sich eine wesentlich größere prozentuale Homologie, vergleicht man ihre genomische Nukleotidsequenzen. Die Werte liegen hier durchweg über 75 %.

**Tabelle 1**

| Homologievergleich zwischen MPV5180/91 und den Konsensus-Sequenzen von HIV 1, HIV 2 sowie der ANT70-Sequenz | | |
|---|---|---|
| Genart | % Homologie | (genähert) |
| LTR | HIV-1 | 67 % |
| | HIV-2 | 51 % |
| | ANT 70 | 82 % |
| GAG | HIV-1 | 70 % |
| | HIV-2 | 62 % |
| | ANT 70 | 89 % |
| POL | HIV-1 | 74 % |
| | HIV-2 | 66 % |
| | ANT 70 | 90 % |
| VIF | HIV-1 | 68 % |
| | HIV-2 | 42 % |
| | ANT 70 | 87 % |
| ENV | HIV-1 | 53 % |
| | HIV-2 | 49 % |
| | ANT 70 | 81 % |
| NEF | HIV-1 | 54 % |
| | HIV-2 | 45 % |
| | ANT 70 | 83 % |
| total | HIV-1 | 65 % |
| | HIV-2 | 56 % |

Aufgrund dieser deutlichen Sequenzunterschiede und aufgrund der Tatsache, daß die Genomorganisation der eines HIV 1-Virus entspricht, wurde das Isolat MVP5180/91 einem neuen HIV 1-Subtyp, dem Subtyp 0 zugeordnet (Myers et al., 1993, Human Retroviruses and AIDS 1993. A compilation and analysis of nucleic acid and aminoacid sequences. Los Alamos National Laboratory, Los Alamos, USA). Der einzige weitere, bisher bekannte Vertreter dieses Subtyps ist das oben zitierte Isolat ANT70.

Der sichere Nachweis einer HIV-Infektion ist heute insbesondere im Blutspendewesen von Interesse. Im Hinblick auf die Virussicherheit von Blut und Blutprodukten wurde die immunchemische Testung von Einzelspenden auf HIV 1-Antikörper in Blutbanken obligatorisch, nachdem 1985 spezifische Anti-HIV 1-Tests verfügbar wurden. Nachdem 1986 HIV 2 entdeckt wurde, stellte sich heraus, daß HIV 2-spezifische Antikörper mit etablierten HIV 1-Tests nicht so zuverlässig nachgewiesen werden konnten wie Anti-HIV 1 mit entsprechenden HIV 1-Antikörpertests. Seit 1989 sind nun sogenannte Kombinationstests verfügbar, die den gleichzeitigen nicht differenzierenden Nachweis von Anti-HIV 1 und Anti-HIV 2 gestatten. Der Mehrzahl kommerzieller Ausführungen der Anti-HIV 1/-HIV 2 Kombinationstests liegen gentechnologisch bzw. peptidsynthetisch hergestellte HIV-Antigene zugrunde.

Bei diesen diagnostischen Tests wird eine Serumprobe der zu untersuchenden Person mit den Proteinketten von einem oder mehreren Proteinen, Peptiden oder Glykoproteinen (die in eukaryontische Zellinien exprimiert werden können) eines Virus oder Teilen davon zusammengebracht. Bevorzugte Testverfahren schließen die Immunfluoreszenz oder immunenzymatische Testverfahren (z.B. ELISA, Immunoblot) ein.

Bei den immunenzymatischen Tests kann beispielsweise Antigen, das von MVP5180/91 oder einer Variante davon stammt, an den Wänden von Mikrotitrationsplatten gebunden werden. Die dabei verwendete Dosierung hängt vom Testsystem und der Behandlung der Mikrotitrationsplatte wesentlich ab. Dann wird Serum bzw. Serumverdünnungen, die von der zu untersuchenden Person stammen, in die Löcher der Mikrotiterplatten gegeben. Nach einer bestimmten Inkubationszeit wird die Platte gewaschen. Spezifische Immunkomplexe werden dadurch nachgewiesen, daß Antikörper, die spezifisch an menschliche Immunglobuline binden und die vorher mit einer Anzeigekomponente, beispielsweise Meerrettichperoxidase oder alkalischer Phosphatase usw. gekoppelt wurden, an diese binden. Diese spezifischen Immunkomplexe können alternativ auch mittels dem Fachmann bekannten Sandwich-Verfahren durch markiertes Antigen detektiert werden. Diese Enzyme können ein farbloses Substrat in ein stark gefärbtes Produkt umwandeln, und an der Stärke der Färbung kann dann das Vorhandensein von spezifischen Anti-HIV-Antikörpern abgelesen werden.

Da MVP5180/91 zu anderen HIV 1-Isolaten, die nicht dem Subtyp 0 angehören und auch gegenüber HIV 2-Isolaten eine relativ geringe Homologie aufweist, stellt sich die Frage, ob die in kommerziellen Anti-HIV 1/-HIV 2-Testen verwendeten Antigene dazu geeignet sind, auch Antikörper nachzuweisen, die auf Infektionen mit MVP5180/91 oder einem mit MVP5180/91 eng verwandten Virus zurückgehen.

Überraschenderweise wurde gefunden, daß HIV-Antikörper in Seren von Immunschwäche-Patienten, die mit leistungsfähigen kommerziell erhältlichen Anti-HIV 1/2 Screeningtests unzureichend erfaßt werden, mit Immunoassays, die auf MVP5180/91-Antigen basieren, zuverlässig nachgewiesen werden. In der vorliegenden Erfindung wird weiterhin gezeigt, daß bei gleichzeitiger Beschichtung von Mikrotitrationsplatten mit HIV 1 und MVP5180/91 abgeleiteten Peptiden der simultane Nachweis von Antikörpern, die gegen diese Viren gerichtet sind, ermöglicht wird. Gegenstand der Erfindung sind weiterhin MVP5180/91-abgeleitete rekombinante Antigene, die ebenfalls den Nachweis von MVP5180/91 bzw. Serotyp 0-spezifischer Antikörper erlauben. Darüber hinaus werden Peptide beschrieben, die eine selektive spezifische Diskriminierung von Sub typ 0- und "Nicht"-Sub typ 0-spezifischen HIV-Antikörpern ermöglichen.

Bei der Diagnostik von HIV-Infektionen ist es unumgänglich, eine höchstmögliche Sicherheit durch den diagnostischen Test dahingehend zu erhalten, ob eine HIV-Infektion vorliegt oder nicht. Besonders bei Blutbanken ist es essentiell, daß Infektionen des HIV-Virus mit möglichst 100 %iger Sicherheit ausgeschlossen werden können. Bereits eine geringfügige Erhöhung der Sicherheit hat enorme Bedeutung, da bereits durch die Identifizierung einer Blutkonserve als HIV-positiv die Infektion eines Menschen mit dem HIV-Virus durch Bluttransfusion ausgeschlossen werden kann.

Gegenstand der vorliegenden Erfindung sind daher solche Peptide, die insbesondere zum diagnostischen Nachweis von Retroviren vom HIV-Typ geeignet sind. Diese Peptide sind dadurch gekennzeichnet, daß sie eine aufeinanderfolgende Aminosäuresequenz von 20 bis 30 Aminosäuren aufweisen und ausgewählt sind aus der Aminosäuresequenz: wobei X die Bedeutung C oder S hat.

In einer bevorzugten Ausführungsform sind die Peptide ausgewählt aus der Sequenz: Die oben angegebene Aminosäuresequenz ist dargestellt durch den Einbuchstabencode, wobei die einzelnen Buchstaben folgende Bedeutung haben: A = Alanin, R = Arginin, N = Asparagin, D = Asparaginsäure, C = Cystein, Q = Glutamin, E = Glutaminsäure, G = Glycin, H = Histidin, I = Isoleucin, L = Leucin, K = Lysin, M = Methionin, F = Phenylalanin, P = Prolin, S = Serin, T = Threonin, W = Tryptophan, Y = Tyrosin und V = Valin.

Wenn die Aminosäuresequenz im sogenannten Dreibuchstabencode dargestellt wird, ergibt sich folgende Sequenz: wobei X die Bedeutung Cys oder Ser haben kann. In besonders bevorzugter Ausführungsform sind die Bedeutungen von X in einem Peptid gleich, d.h. es liegt zweimal Cystein oder zweimal Serin vor.

Unter immunologisch aktiven Peptiden werden solche Peptide verstanden, die mit Antikörpern gegen HIV-Viren, die sich im Blut von Patienten oder Blutspendern befinden können, reagieren. Üblicherweise enthalten daher immunologisch aktive Peptide wenigstens ein Epitop, das eine Bildung von Antikörpern verursacht.

Es wurde herausgefunden, daß sich ein für die Diagnostik hauptsächlich relevantes Epitop in dem Bereich XKGKLIX befindet.

Die erfindungsgemäßen Peptide weisen rechts und/oder links von diesem Epitop noch einen zusätzlichen Bereich auf, der zu der entsprechenden Sequenz des Virus MVP5180 homolog ist. Dieser Bereich hat vor allem dann Bedeutung, wenn die Peptide in diagnostischen Tests eingesetzt werden. Werden die Peptide an eine Festphase gebunden, wie das beispielsweise beim ELISA-Test der Fall sein kann, dann ist es vorteilhaft, wenn das Peptid eine Aminosäuresequenz links von dem Epitop aufweist. Wenn Konjugationen stattfinden, dann ist es vorteilhaft, wenn die Peptide eine zu MVP5180 homologe Sequenz rechts von dem Hauptepitop aufweisen.

Die erfindungsgemäßen Peptide weisen eine Länge von etwa 20 bis etwa 30 Aminosäuren auf. Im Rahmen der vorliegenden Erfindung sind die folgenden Peptide besonders bevorzugt: Neben der zu MVP5180/91 homologen Aminosäuresequenz können die erfindungsgemäßen Peptide an einem oder beiden Enden des Peptids weitere Aminosäuren aufweisen, die für bestimmte Funktionen wichtig sind. Es kann sich hierbei um Aminosäuren handeln, die beispielsweise die Bindungen des Peptids an Festphasen erleichtern. Wenn die Peptide rekombinant hergestellt werden, können die Peptide auch Aminosäuren enthalten, die durch die Art der rekombinanten Herstellung entstehen.

Hergestellt werden können diese Peptide nicht nur mit Hilfe der rekombinanten Technologie, sondern auch durch synthetische Methoden. Ein geeigneter Herstellungsweg ist die Festphasensynthese vom Merrifield-Typ. Eine weitere Beschreibung dieser Technik und anderer im Stand der Technik bekannter Verfahren kann in der Literatur gefunden werden, z.B. M. Bodansky et al., Peptide Synthesis, John Wi ley & Sons, 2nd Edition 1976.

Gegenstand der vorliegenden Erfindung sind auch isolierte DNA-Fragmente, die komplementär sind zu den für die Peptide kodierenden DNA-Sequenzen. Derartige isolierte DNA-Fragmente können in der an sich bekannten Polymerase-Kettenreaktion (PCR) eingesetzt werden.

Wie in den nachfolgenden Beispielen gezeigt, sind die erfindungsgemäßen Peptide besonders vorteilhaft verwendbar in Testkits zum Nachweis von Antikörpern gegen Immunschwäche verursachende Viren. Eingesetzt wird dabei wenigstens ein erfindungsgemäßes Peptid.

Bei den Testkits kann es sich um sogenannte Western Blots handeln, in einer besonders bevorzugten Ausführungsform handelt es sich aber um sogenannte ELISA-Tests oder um Fluoreszenz-Antikörpernachweistests. Darüber hinaus eignen sich die erfindungsgemäßen Peptide auch zur Herstellung von Impfstoffen, insbesondere gegen Infektionen von HIV-Viren des Subtyps O.

Die nachfolgenden Beispiele stellen Ausführungsformen der Erfindung dar, ohne sie jedoch zu beschränken.

### Beispiel 1:

### Indirekter Immunoassay zum HIV-Nachweis von Subtyp O-spezifischen Antikörpern

### Beispiel 1a:

### Synthese des erfindungsgemaßen Peptides MVP 601-623 sowie des HIV 1-Peptids HIV 601-623

Die Synthese von MVP 601-623 (siehe Tabelle 2) aus dem Transmembranprotein gp41 von MVP5180 erfolgt nach BARANI, G. und MERRIFIELD, R.B. in "The Peptides, Analysis, Synthesis and Biology", Vol. 2, Academic Press, Ed. Erhard Gross, Johannes Meyerhofer. Die analytische Reinheit nach HPLC beträgt 81 %.

Ebenfalls nach der Merrifield-Methode wird das Referenz-Peptid HIV 601-623 (siehe Tabelle 2) synthetisiert. Das Rohpeptid wird über HPLC gereinigt. Die Reinheit beträgt 87 %.

Die Tabelle 2 zeigt den im rekombinanten Plasmid pSEM 41/3-III exprimierten Sequenzbereich aus gp41 von MVP5180, im Vergleich zur korrespondierenden Sequenz des HIV 1-Isolates ARV-2. Bei dem mit HIV gekennzeichneten Peptid handelt es sich um HIV 1-Isolat-abgeleitete Sequenzen. Bei den mit MVP bezeichneten Peptiden handelt es sich um MVP5180-abgeleitete Sequenzen. Die Numerierung der Sequenzen bezieht sich auf die Angaben zu der HIV 1 BH10 env-Sequenz in Rattner et al., Nature, 313: 277-284.

### Beispiel 1 b:

### Herstellung der Peptid-Lösungen sowie Beschichtung von Mikrotitrationsplatten mit diesen Peptiden

Die Peptide MVP 601-623 und HIV 601-623 aus Beispiel 1 a werden in einer Konzentration von 6 mg/ml in 50 % (v/v) Essigsäure gelöst.

Die Stammlösungen werden in 0,10 M Natriumbicarbonat (pH 9,6) so verdünnt, daß die Konzentration der Polypeptide 1 µg/ml beträgt.

Jeweils 100 µl der verdünnten Lösung werden in die Vertiefungen von Mikrotitrationsplatten, Typ B der Firma Nunc, Roskilde, Dänemark gegeben. Die gefüllten Testplatten wurden 18 Stunden bei 20°C inkubiert. Anschließend wurden die Lösungen abgesaugt und die Vertiefungen 3-4 mal mit 300 µl einer Lösung von 10 g/l Rinderserumalbumin in phosphatgepufferter physiologischer Kochsalzlösung (PBS, pH 7,4) gespült und die Testplatten anschließend über Kieselgel bei 20°C getrocknet.

### Beispiel 1 c:

### Herstellung eines Peroxidase-markierten Antikörpers gegen humanes Immunglobulin der IgG-Klasse (h-IgG) sowie TMB-Substrat für die Detektion

Monoklonale Antikörper gegen h-IgG wurden gemäß der Methode von KOEHLER und MILSTEIN hergestellt (Nature 256, 495, 1975), wobei verschiedene monoklonale Antikörper mit der gleichen Antigen-Spezifität mit der von STÄHLI et al. (J. of Immunological Methods 32, 297-304, 1980) beschriebenen Methode identifiziert wurden.

Nach gelchromatographischer Reinigung und Dialyse gegen PBS-Puffer, pH 7,4 wurde die monoklonale Antikörper-Fraktion (4 mg Protein/ml) mit N-gamma-Maleimidobutyloxysuccinimid (GMBS), nach TANAMORI et al. (J. Immunol. Meth. 62, 123-131, 1983) umgesetzt. Parallel dazu wurde 2-Iminothiolanhydrochlorid (Fa. Sigma, Kat.-Nr. I 6256) mit Meerrettich-Peroxidase (POD, Fa. Boehringer Mannheim, Kat.-Nr. 413470) nach KING et al. (Biochem. 17, 1499-1506, 1978) umgesetzt. Aus dem GMBS-Antikörper-Konjugat und dem Iminothiolan-POD-Konjugat wurde ein Antikörper-POD-Konjugat, wie von TANAMORI et al. (supra) beschrieben, hergestellt.

Die erhaltene Lösung des IgG-POD-Konjugats hatte einen Proteingehalt von 360 µl/ml. Das Verhältnis von POD zu IgG ergab 2,8. Die Lösung wurde anschließend auf 500 ng/ml IgG-POD mit einer Lösung von 50 ml/l foetalem Kälberserum (FKS, Fa. Biochrom KG, Berlin), 5 g/l Polyoxiethylen-(20)sorbitan Monolaureat (Tween 20) in PBS verdünnt und erhielt die Bezeichnung Anti-IgG/POD-Konjugat. Für die Verwendung im ELISA wurde das Anti-IgG/POD-Konjugat mit Tris-Puffer (pH 7,4, enthaltend 0,5 % Tween 20) 1 : 100 bis 1 : 20.000 verdünnt, um einheitlich eine 1 : 26-Endverdünnung in Konjugat-Puffer (0,1 M 1-Amino-2-(hydroxy-methyl)-1,3-propandiol (Tris), 0,1 M Kochsalz (NaCl) sowie 0,1 % Tween 20, pH 8,4) herzustellen.

Zum Nachweis von Anti-Human/IgG-POD wurde ein Substratsystem oder eine Substratzubereitung aus Wasserstoffperoxid und Tetramethylbenzidin (TMB) verwendet, welche aus zwei Stammlösungen wie folgt hergestellt wurde.

Stammlösung 1: TMB-Dihydrochlorid wurde unter Rühren in einer Konzentration von 5 g/l, (16 mmol/l) in bidestilliertem Wasser gelöst und mit 5 N Salzsäure auf pH 1,5 eingestellt. Zu dieser Lösung wurde Penicillin G unter Rühren bis zu einer Endkonzentration von 200 mg/l (0,56 mmol/l) zugesetzt.

Stammlösung 2: Zu 900 ml bidestilliertem Wasser wurde 1,4 ml Eisessig, 1,5 ml 1 N NaOH und 250 mg (3 mmol) H₂O₂ als Harnstoff-Wasserstoffperoxid-Addukt zugesetzt. Nach vollständigem Lösen wurde mit bidestilliertem Wasser auf 1 l aufgefüllt.

TMB-Substratzubereitung: Ein Volumenteil der Stammlösung 1 und 10 Volumenteile der Stammlösung 2 wurden miteinander vermischt.

### Beispiel 1 d:

### Bestimmung von humanen Antikörpern der Immunglobulin G-Klasse gegen MVP5180 im ELISA mit dem erfindungsgemäßen Peptid

50 µl Serum oder Plasma wurden zu 50 µl Probenpuffer, enthaltend 0,3 M Tris, 0,3 M NaCl, 20 % Rinderserum und 0,1 % Tween 20 in Vertiefungen von beschichteten Mikrotiterplatten, hergestellt nach Beispiel 1 b, gegeben. Nach 30minütiger Inkubation bei 37°C wurden die Testlösungen abgesaugt und die Vertiefungen jeweils fünfmal mit Waschpuffer enthaltend 1 g/l Tween 20 in PBS gewaschen. Danach gab man jeweils 100 µl Konjugat (nach Beispiel 1 c) in die Vertiefungen, wobei bevorzugt eine Vorverdünnung von 1 : 3000 in Tris-Puffer (pH 7,4, 0,5 % Tween 20) und eine Endverdünnung von 1 : 26 in Konjugatpuffer gewählt wurde. Nach einer Inkubation über 30 Minuten bei 37°C wurde der Inhalt der Vertiefungen abgesaugt und wiederum jeweils fünfmal gewaschen. Anschließend wurde in jede Vertiefung 100 µl TMB-Substratzubereitung gegeben, 30 Minuten bei 20-22°C inkubiert und die Reaktion durch Zugabe von 100 µl 1-normaler Schwefelsäure gestoppt. Die Extinktion der gefärbten Lösung wurde bei einer Wellenlänge von 450 nm (E450) gegen einen Leerwert von PBS gemessen.

In Tabelle 3 werden die Reaktivitäten von Western Blot Anti-HIV 1 negativen und Western Blot Anti-HIV 1 positiven Proben (alle von Blutspendern aus Kamerun) auf Mikrotitrationsplatten verglichen, die zum einen mit dem synthetischen Peptid MVP 601 - 623 und zum anderen mit HIV 601 - 623 beschichtet sind.

**Tabelle 3**

| Status nach Western Blot | Proben I.D. | Signal/Cut off MVP 601-623 (erfindungsgem. Reagenz) | Signal/Cut off HIV 601-623 (Referenz) |
|---|---|---|---|
| Anti-HIV negativ | 16749 | 0,1 (-) | 0,2 (-) |
| | 16750 | 0,1 (-) | 0,1 (-) |
| Anti-HIV positiv | 17038 | > 6 (+) | 0,7 (-) |
| | 17041 | 0,8 (-) | 3,0 (+) |
| | 16717 | > 6 (+) | > 6 (+) |
| | 16748 | > 6 (+) | > 6 (+) |
| cut off | | 0,400 | 0,400 |

Aus Tabelle 3 wird ersichtlich, daß manche Proben (16717 und 16748) deutlich positiv in beiden Assays reagieren, andere reagieren jedoch (17038) nur mit dem erfindungsgemäßen MVP-Peptid.

### Beispiel 2:

### Immunometrischer Immunoassay zum Nachweis von Serotyp O-spezifischen HIV-Antikörpern

### Beispiel 2 a:

### Herstellung des MVP 601-623-Peptid POD-Konjugates

10 mg des erfindungsgemäßen Peptides MVP 601 - 623 (Beispiel 1 a) werden in 1 ml Eisessig/Wasser (50 : 50, v/v) gelöst. Nach Neutralisieren mit 5 N Natronlauge wird mit einem 10 fach molaren Überschuß an GMBS versetzt und 1 h bei Raumtemperatur inkubiert. Das nicht umgesetzte GMBS wird durch Gelfiltration (Sephadex G-25) mit 0,1 M Natriumphosphat/5 mmol/l Nitrilotriessigsäure, pH 6,0 abgetrennt. 10 mg Meerrettich-Peroxidase (POD) werden in 5 ml 10 mmol/l Natriumphosphat, 100 mmol/l NaCl, pH 8,0 mit 100fach molarem Überschuß an 2-Iminothiolan 1 h bei Raumtemperatur inkubiert. Anschließend wird freies Modifizierungsreagenz über Gelchromatographie (Sephadex G-25) mit 0,1 M Natriumphosphat/5 mmol/l NTA, pH 6,0 entfernt. Beide Eluate (SH-aktivierte Peroxidase und Maleimidmodifiziertes HIV 1-Peptid) werden vereinigt und über Nacht bei Raumtemperatur inkubiert. Nach Abstoppen mit 1/19 Vol. 0,1 M N-Ethyl-Maleimid wird das Konjugat durch Gelchromatographie (Sephadex G-25) von nicht abreagiertem HIV 1-Peptid gereinigt. Nach Ankonzentrieren (2 mg/ml) wird das Peptid-Peroxidase-Konjugat bei -20°C gelagert.

### Beispiel 2 b:

### Immunometrischer Immunoassay zum Nachweis von Anti-MVP-Antikörpern

Ein Enzymimmunoassay zum Nachweis von Anti-HIV-Antikörpern wird wie folgt durchgeführt: In den Vertiefungen einer mit HIV-Peptiden beschichteten Testplatte werden 25 µl Probenpuffer (0,3 M Tris/HCl, 1 % Albumin, 2 % Tween 20, pH 7,2) mit 100 µl Human-Serum für 30 min bei 37°C inkubiert. Nach 4maligem Waschen mit 50 mmol/l PBS, 0,1 % Tween 20 werden 100 µl des nach Beispiel 2 a hergestellten HIV-Peptid-Peroxidase-Konjugates (1 : 1000 in 0,1 M Tris/HCl, 1 % Albumin, 2 % Pluronic F 64, pH 8,1) einpipettiert.

Mit 4 weiteren Waschschritten wird die 30minütige Inkubation (+37°C) beendet. Die gebundene Peroxidase-Aktivität, die direkt mit der Anzahl der gebundenen HIV 1-spezifischen
Antikörper korreliert, wird durch Zugabe von H₂O₂/Tetramethylbenzidin (Behringwerke AG, Marburg, FRG) bestimmt.

### Beispiel 2 c:

### Anwendung des erfindungsgemaßen Reagenzes

Western Blot charakterisierte Anti-HIV-negative und -positive Proben (siehe auch Beispiel 1) werden im Immunoassay nach Beispiel 2 b untersucht.

Die Resultate (Signal/Cut off) dieser Untersuchung finden sich in der Tabelle 4 sowie vergleichende Untersuchungen gegen einen kommerziellen Anti-HIV-Assay der 3. Generation (immunometrisches Testprinzip).

**Tabelle 4**

| Probenstatus nach Western Blot | Proben I.D. | Signal/Cut off MVP immunometr. (erfindungsgem. Reagenz) | Signal/Cut off Anti-HIV (3. Gen. )-Assay Referenz |
|---|---|---|---|
| Anti-HIV negativ | 16749 | 0,1 (-) | 0,1 (-) |
| | 16750 | 0,1 (-) | 0,1 (-) |
| Anti-HIV positiv | 17038 | > 16,6 (+) | 0,8 (-) |
| | 17041 | 0,6 (-) | 9,5 (+) |
| | 16717 | > 16,6 (+) | 14,1 (+) |
| | 16748 | > 16,6 (+) | 9,6 (+) |
| cut off | | 0,150 | 0,141 |

Bei diesem Vergleich zeigt sich, daß die unterschiedlichen Antigene auch bei gleichem Assaytestprinzip speziell mit den Proben 17038 und 17041 unterschiedlich erkannt werden. Das erfindungsgemäße Antigen zeigt sehr deutlich die Anwesenheit von HIV-Antikörpern in der Probe 17038, während der kommerzielle Referenzassay ungenügend reagiert.

### Beispiel 3:

### Immunoassay zum selektiven Nachweis von Serotyp 0-spezifischen HIV-Antikörpern

### Beispiel 3 a:

### Synthese der erfindungsgemäßen Peptide sowie deren Referenzpeptide

Nach der Methode aus Beispiel 1 a werden folgende 4 Peptide synthetisiert: Nach HPLC-Reinigung der 4 Rohpeptide erhält man Reinheiten von 81 % - 89 %.

### Beispiel 3 b:

### Beschichtung und Durchführung

Die 4 nach Beispiel 3 a hergestellten und gereinigten Peptide werden nach Beispiel 1 b gelöst und auf Mikrotitrationsplatten beschichtet. Entsprechend Beispiel 1 d wird ein Assay durchgeführt.

### Beispiel 3 c:

### Anwendung des erfindungsgemäßen Reagenzes

Die aus den Beispielen 1 und 2 bekannten Proben werden entsprechend Beispiel 3 b im indirekten Antikörpertest sowohl auf den erfindungsgemäßen Peptiden MVP 591-616 C und

MVP 591-616 S sowie auf den Referenzpeptiden getestet. Die Resultate dieser Untersuchungen sind in Tabelle 5 aufgelistet.

**Tabelle 5**

| Status nach Western Blot | Proben I.D. | Signal/Cut off | | Signal/Cut off | |
|---|---|---|---|---|---|
| | | MVP 591-616 C | MVP 591-616 S | HIV 591-616 C | HIV 591-616 S |
| | | erfindungsgem. Peptid | | Referenz | |
| Anti-HIV negativ | 16749 | 0,6 (-) | 0,6 (-) | 0,5 (-) | 0,5 (-) |
| | 16750 | 0,4 (-) | 0,5 (-) | 0,1 (-) | 0,8 (-) |
| Anti-HIV positiv | 17038 | 14,2 (+) | 5,6 (+) | 5,3 (+) | 0,1 (-) |
| | 17041 | 0,3 (-) | 0,3 (-) | 5,1 (+) | 2,8 (+) |
| | 16717 | > 16,6 (+) | 0,7 (-) | > 8,3 (+) | > 8,3 (+) |
| | 16748 | 16,2 (+) | 0,7 (-) | > 8,3 (+) | > 8,3 (+) |
| cut off | | 0,150 | 0,150 | 0,300 | 0,300 |

Wie aus Tabelle 5 ersichtlich, ist eine selektive spezifische Diskriminierung von Serotyp 0- und "Nicht"-Serotyp 0-spezifischen HIV-Antikörpern möglich, wenn die Signal/cut off-Werte des Assay MVP 591-616 S mit dem Assay HIV 591-616 S verglichen werden.

### Beispiel 4:

### Immunoassay zum simultanen Nachweis von Subtyp A-E-spezifischen und Subtyp O-spezifischen HIV-Antikörpern

### Beispiel 4 a:

### Herstellung von Peptid-Lösungen und Beschichtung von Mikrotitrationsplatten

Die nach Beispiel 1 a hergestellten Peptide MVP 601-623 und HIV 601-623 werden in einer Konzentration von 6 mg/ml in 50 % (v/v) Essigsäure gelöst.

Die Stammlösungen werden auf Volumenbasis in unterschiedlichen Verhältnissen gemischt und in 0,10 M Natriumcarbonat (pH 9,6) so verdünnt, daß die Gesamtkonzentration der Peptide zwischen 0,125 und 2 µg/ml beträgt. Wie in Beispiel 1 b werden diese Lösungen in Mikrotitrationsplatten gegeben und die Antigene beschichtet.

### Beispiel 4 b:

### Durchführung des Immunoassays und Resultate

Nach Beispiel 1 c und 1 d wird ein Immunoassay durchgeführt.

Die Resultate sind in Tabelle 6 zusammengefaßt.

**Tabelle 6**

| Status nach Western Blot | Proben I.D. | Signal/Cut off MVP 601-623 | Signal/Cut off HIV 601-623 | Signal/Cut off MVP 601-623 *und* HIV 601-623 (erfindungsgemäß) |
|---|---|---|---|---|
| Anti-HIV negativ | 16749 | 0,2 (-) | 0,2 (-) | 0,2 (-) |
| | 16750 | 0,5 (-) | 0,2 (-) | 0,2 (-) |
| Anti-HIV positiv | 17038 | > 10 (+) | 0,4 (-) | > 10 (+) |
| | 17041 | 0,5 (-) | 2,5 (+) | 4,7 (+) |
| | 16717 | > 10 (+) | > 10 (+) | > 10 (+) |
| | 16748 | > 10 (+) | > 7 (+) | > 10 (+) |
| cut off | | 0,250 | 0,250 | 0,250 |

### Beispiel 5:

### Immunoassay zum Nachweis von Subtyp O-spezifischen HIV-Antikörpern mittels rekombinanter Antigene

### Beispiel 5 a:

### Konstruktion des Plasmids pSEM 41/3-III

Die serodiagnostische Bedeutung des MVP5180/91 gp41-Bereiches sollte untersucht werden. Zu diesem Zweck wurde ein rekombinanter Expressionsklon konstruiert, der einen Teilbereich aus gp41 von MVP5180 enthält. Die Methodik zur Konstruktion solcher Plasmide ist bekannt (Sambrook, Fritsch, Maniatis, Molecular Cloning, A Laboratory Manual, 2nd Edition, Cold Spring Harbor Laboratory Press, 1989).

Ein geeignetes DNA-Stück aus gp41 wurde mittels PCR (Polymerase Chain Reaction, US-A-4.683195 und 4.683202) erhalten. Dazu wurden folgende Primer eingesetzt:
1 A: und
1 B:

Als Template wurden 0,1 µg Plasmid-DNA pSP4 eingesetzt (DE 4318184). Die Bedingungen für die PCR waren
1. Initial Denaturierung: 94°C, 3 min,
2. Amplifikation: 1,5 min., 94°C, 1 min., 56°C und 1 min. 72°C
für 30 Zyklen. Die Nukleotid- und Pufferkonzentration sowie Taq-Polymerase wurden nach Vorgaben des Herstellers eingesetzt (Fa. Perkin Elmer).

Anschließend wurde die amplifizierte DNA mit den Restriktionsendonukleasen Asp 718 und XbaI für 1 Stunde bei 37°C verdaut und die DNA in einem 1 % Agarosegel aufgetrennt. Die DNA-Bande der Größe von 440 bp wurde aus dem Gel ausgeschnitten, elektroeluiert, phenolisiert und mit Ethanol präzipitiert, getrocknet und in 5 µl H₂O resuspendiert.

0,5 µg des gelösten DNA-Amplifikates wurden mit 0,5 µg des Asp718-XbaI verdauten Expressionsvektors pSEM 3 (Knapp et al., Biotechniques 8, 280-281 [1990]) ligiert (2 Weiss-Units Lambda T4 Ligase, 12 Std. bei 15°C) und in E.coli XL1 Blue (Fa. Stratagene) transformiert. Der daraus resultierende Klon mit dem rekombinanten Plasmid pSEM 41/3-III exprimiert das MvP5180-gp41 spezifische Peptid als Fusionsprotein mit einem Fragment der E.coli-β-Galactosidase.

In Tabelle 2 ist die exprimierte MVP5180 Sequenz abgebildet.

### Beispiel 5 b:

### Expression und Reinigung des MvP 41/3-III-Fusionsproteins

Escherichia coli XL1 Blue, transformiert mit dem Plasmid pSEM 41/3-III (nach Beispiel 5 a), wurde in Luria-Broth-Medium kultiviert und bei einer optischen Dichte von 0,5 mit 1 mM Isopropylthiogalactosid induziert. Nach drei Stunden wurden die Zellen abzentrifugiert, mit 100 mM Natriumphosphat-Puffer, 10 mM MgCl₂, pH 7,5 gewaschen und nach Zentrifugation, 10 Minuten, 5000 x g, im gleichen Puffer resuspendiert. Nach Zugabe von RNase und DNase wurde die Zellsuspension mit einem Hochdruckhomogenisator bei 1000 bar aufgeschlossen und das Homogenat zentrifugiert (20 Minuten, 80.000 x g, 4°C). Das Sediment enthielt die Einschlußkörper (Inclusion Bodies) und wurde in 50 mM Tris-HCl pH 8.0 und 0.5 % Desoxycholat resuspendiert und nochmals zentrifugiert (20 Minuten, 100.000 x g, 4°C). Das gewonnene Sediment wurde in 3 M Harnstoff, 20 mM Tris-HCl, 0.5 mM Phenylmethysulfonylfluorid (PMSF) resuspendiert und nochmals zentrifugiert (20 Minuten, 100.000 x g, 4°C).

Das jetzt zweimal gewaschene Sediment wurde anschließend 1 Stunde in 5 M Guanidin-HCl, 10 mM Tris-HCl, 5 mM Ethylendiamintetraacetat (EDTA), 0.5 mM PMSF und 100 mM Dithiothreit inkubiert. Nach Zentrifugation (20 Minuten, 100.000 x g, 4°C) wurde der Überstand, der das solubilisierte MVP 41/3-III-Protein enthielt, durch Gelfiltration auf TSK-HW-55 S (Fa. Merck, Darmstadt) in 5 M Guanidin-HCl, 10 mM Tris-HCl, 5 mM EDTA, pH 8.0, chromatographisch gereinigt. Die produkthaltigen Fraktionen wurden durch Elektrophorese identifiziert, vereinigt und durch Umpufferung in 5 M Harnstoff 10 mM Tris-HCl, 5 mM EDTA, pH 8.0 überführt.

### Beispiel 5 c:

### Immunoassay zum Nachweis von Subtyp O-spezifischen HIV-Antikörpern

Das nach Beispiel 5 b gereinigte erfindungsgemäße rekombinante Antigen MVP 41/3-III wird in 0,1 M Natriumcarbonat (pH 9,6) so verdünnt, daß die Konzentration des Proteins 0,5 µg/ml beträgt.

Wie in Beispiel 1 b beschrieben, wird das Antigen beschichtet und der so hergestellte Assay wie in Beispiel 1 d durchgeführt.

### Beispiel 5 d:

### Resultate des rekombinanten Antigens im Immunoassay

Die Ergebnisse der nach Beispiel 5 c untersuchten Proben sind in Tabelle 7 zusammengefaßt:

**Tabelle 7**

| Status nach Western Blot | Proben I.D. | Signal/Cut off MVP 41/3-III (erfindungsgemäßes (Reagenz) |
|---|---|---|
| Anti-HIV negativ | 16749 | 0,6 (-) |
| | 16750 | 0,5 (-) |
| Anti-HIV positiv | 17038 | 4,2 (+) |
| | 17041 | 2,1 (+) |
| | 16717 | 3,2 (+) |
| | 16748 | 3,8 (+) |
| cut off | | 0,500 |

Mit diesen Ergebnissen wird deutlich, daß auch rekombinante Proteine aus MVP5180-gp41, die den erfindungsgemäßen Bereich enthalten, sehr gute Antigene sowohl für die Detektion von Subtyp O- als auch für "Nicht"-Serotyp O-spezifischen HIV-Antiseren geeignet sind.

### SEQUENCE LISTING

(1) GENERAL INFORMATION:
   APPLICANT:
      (A) NAME: Behringwerke Aktiengesellschaft
      (B) STREET:
      (C) CITY: Marburg
      (E) COUNTRY: Germany
      (F) POSTAL CODE (ZIP): 35001
   TITLE OF INVENTION:
      Von MvP5180 abgeleitete Peptide
   NUMBER OF SEQUENCES: 10
   COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PADAT Sequenzmodul, Version 1.0
(2) INFORMATION FOR SEQ ID NO: 1:
   (i) SEQUENCE CHARACTERISTICS:
      (A) LENGTH: 48 amino acids
      (B) TYPE: amino acid
      (C) STRANDEDNESS: unknown
      (D) TOPOLOGY: unknown
   (ii) MOLECULE TYPE: Peptide
   (v) FRAGMENT TYPE: internal
   (xi) SEQUENCE DESCRIPTION: SEQ ID NO: 1:
(2) INFORMATION FOR SEQ ID NO: 2:
   (i) SEQUENCE CHARACTERISTICS:
      (A) LENGTH: 32 amino acids
      (B) TYPE: amino acid
      (C) STRANDEDNESS: unknown
      (D) TOPOLOGY: unknown
   (ii) MOLECULE TYPE: Peptide
   (v) FRAGMENT TYPE: internal
   (xi) SEQUENCE DESCRIPTION: SEQ ID NO: 2:
(2) INFORMATION FOR SEQ ID NO: 3:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: Peptide
   (v) FRAGMENT TYPE: internal
   (xi) SEQUENCE DESCRIPTION: SEQ ID NO: 3:
(2) INFORMATION FOR SEQ ID NO: 4:
   (i) SEQUENCE CHARACTERISTICS:
      (A) LENGTH: 26 amino acids
      (B) TYPE: amino acid
      (C) STRANDEDNESS: unknown
      (D) TOPOLOGY: unknown
   (ii) MOLECULE TYPE: Peptide
   (v) FRAGMENT TYPE: internal
   (xi) SEQUENCE DESCRIPTION: SEQ ID NO: 4:
(2) INFORMATION FOR SEQ ID NO: 5:
   (i) SEQUENCE CHARACTERISTICS:
      (A) LENGTH: 26 amino acids
      (B) TYPE: amino acid
      (C) STRANDEDNESS: unknown
      (D) TOPOLOGY: unknown
   (ii) MOLECULE TYPE: Peptide
   (v) FRAGMENT TYPE: internal
   (xi) SEQUENCE DESCRIPTION: SEQ ID NO: 5:
(2) INFORMATION FOR SEQ ID NO: 6:
   (i) SEQUENCE CHARACTERISTICS:
      (A) LENGTH: 23 amino acids
      (B) TYPE: amino acid
      (C) STRANDEDNESS: unknown
      (D) TOPOLOGY: unknown
   (ii) MOLECULE TYPE: Peptide
   (v) FRAGMENT TYPE: internal
   (xi) SEQUENCE DESCRIPTION: SEQ ID NO: 6:
(2) INFORMATION FOR SEQ ID NO: 7:
   (i) SEQUENCE CHARACTERISTICS:
      (A) LENGTH: 26 amino acids
      (B) TYPE: amino acid
      (C) STRANDEDNESS: unknown
      (D) TOPOLOGY: unknown
   (ii) MOLECULE TYPE: Peptide
   (v) FRAGMENT TYPE: internal
   (xi) SEQUENCE DESCRIPTION: SEQ ID NO: 7:
(2) INFORMATION FOR SEQ ID NO: 8:
   (i) SEQUENCE CHARACTERISTICS:
      (A) LENGTH: 26 amino acids
      (B) TYPE: amino acid
      (C) STRANDEDNESS: unknown
      (D) TOPOLOGY: unknown
   (ii) MOLECULE TYPE: Peptide
   (v) FRAGMENT TYPE: internal
   (xi) SEQUENCE DESCRIPTION: SEQ ID NO: 8:
(2) INFORMATION FOR SEQ ID NO: 9:
   (i) SEQUENCE CHARACTERISTICS:
      (A) LENGTH: 32 base pairs
      (B) TYPE: nucleic acid
      (C) STRANDEDNESS: single
      (D) TOPOLOGY: unknown
   (ii) MOLECULE TYPE: Primer
   (xi) SEQUENCE DESCRIPTION: SEQ ID NO: 9:
   TGTGTGGTAC CGCAGCGGCA ACAGCGCTGA CG
(2) INFORMATION FOR SEQ ID NO: 10:
   (i) SEQUENCE CHARACTERISTICS:
      (A) LENGTH: 32 base pairs
      (B) TYPE: nucleic acid
      (C) STRANDEDNESS: single
      (D) TOPOLOGY: unknown
   (ii) MOLECULE TYPE: Primer
   (xi) SEQUENCE DESCRIPTION: SEQ ID NO: 10:
   GTGTGTCTAG TTTAGTTATG TCAAACCAAT TC

## Patentansprüche

1. Immunologisch aktives Peptid, dadurch gekennzeichnet, daß es 20 bis 30 aufeinanderfolgende Aminosäuren aufweist, ausgewählt aus der Aminosäuresequenz: wobei X die Bedeutung von C oder S hat.

2. Peptid nach Anspruch 1, dadurch gekennzeichnet, daß die Sequenz ausgewählt ist aus der Aminosäuresequenz wobei X die in Anspruch 1 angegebene Bedeutung hat.

3. Peptid nach Anspruch 1 oder 2, dadurch gekennzeichnet, daß es zum Nachweis von Retroviren vom HIV-Typ geeignet ist.

4. Peptid nach einem der vorhergehenden Ansprüche, dadurch gekennzeichnet, daß es weitere Aminosäuren an einem oder beiden Enden des Peptids aufweist, die nicht von der Sequenz des Virus MVP5180 abgeleitet sind.

5. Peptid nach einem der vorhergehenden Ansprüche, dadurch gekennzeichnet, daß die Bedeutung von C hat und, daß die dem Rest C entsprechenden Cystein-Reste im oxidierten Zustand vorliegen.

6. Peptid nach Anspruch 5, dadurch gekennzeichnet, daß es folgende Aminosäuresequenz aufweist:

7. Peptid nach Anspruch 5, dadurch gekennzeichnet, daß es die Aminosäuresequenz aufweist.

8. Peptid nach einem der Ansprüche 1 bis 4, dadurch gekennzeichnet, daß es folgende Aminosäuresequenz aufweist:

9. Peptid nach einem der vorhergehenden Ansprüche, dadurch gekennzeichnet, daß es synthetisch hergestellt wurde.

10. Peptid nach einem der Ansprüche 1 bis 9, dadurch gekennzeichnet, daß es rekombinant hergestellt wurde.

11. Isoliertes DNA-Fragment, dadurch gekennzeichnet, daß es für ein Peptid nach einem der Ansprüche 1 bis 4 oder 6 bis 8 kodiert.

12. Testkit zum Nachweis von Antikörpern gegen Immunschwäche verursachende Viren, dadurch gekennzeichnet, daß wenigstens ein Peptid gemäß einem der Ansprüche 1 bis 10 eingesetzt wird.

13. Testkit gemäß Anspruch 12, dadurch gekennzeichnet, daß es ein Western Blot ist.

14. Testkit gemäß Anspruch 13, dadurch gekennzeichnet, daß es ein ELISA-Test ist oder ein Fluoreszenz-Antikörpernachweistest ist.

15. Verwendung eines Peptids nach einem der Ansprüche 1 bis 10 zur Herstellung von Impfstoffen.

## Claims

1. An immunologically active peptide, which has 20 to 30 consecutive amino acids, selected from the amino acid sequence: where X has the meaning C or S.

2. A peptide as claimed in claim 1, wherein the sequence is selected from the amino acid sequence where X has the meaning given in claim 1.

3. A peptide as claimed in claim 1 or 2, which is suitable for detecting retroviruses of the HIV type.

4. A peptide as claimed in one of the preceding claims, which has, at one or both ends of the peptide, additional amino acids which are not derived from the sequence of the virus MVP5180.

5. A peptide as claimed in one of the preceding claims, wherein X has the meaning C and the cysteine residues corresponding to the residue C are present in the oxidized state.

6. A peptide as claimed in claim 5, which has the following amino acid sequence:

7. A peptide as claimed in claim 5, which has the amino acid sequence

8. A peptide as claimed in one of claims 1 to 4, which has the following amino acid sequence:

9. A peptide as claimed in one of the preceding claims, which was prepared synthetically.

10. A peptide as claimed in one of claims 1 to 9, which was prepared recombinantly.

11. An isolated DNA fragment, which encodes a peptide as claimed in one of claims 1 to 4 or 6 to 8.

12. A test kit for detecting antibodies against viruses which cause immune deficiency, in which at least one peptide as claimed in one of claims 1 to 10 is employed.

13. A test kit as claimed in claim 12, which is a Western blot.

14. A test kit as claimed in claim 13, which is an ELISA test or a fluorescent antibody detection test.

15. The use of a peptide as claimed in one of claims 1 to 10 for preparing vaccines.

## Revendications

1. Peptide immunologiquement actif, qui se caractérise en ce qu'il présente de 20 à 30 aminoacides successifs, choisis dans la séquence d'aminoacides : où X représente C ou S.

2. Peptide suivant la revendication 1, caractérisé en ce que la séquence est choisie parmi la séquence d'aminoacides : où X a la signification qui lui a été attribuée dans la revendication 1.

3. Peptide suivant la revendication 1 ou 2, caractérisé en ce qu'il convient à la détermination de rétrovirus du type VIH.

4. Peptide suivant l'une quelconque des revendications précédentes, caractérisé en ce qu'il présente d'autres aminoacides à l'une ou aux deux extrémités du peptide, qui ne dérivent pas de la séquence du virus MVP5180.

5. Peptide suivant l'une quelconque des revendications précédentes, caractérisé en ce que X représente C et en ce que les restes de cystéine correspondant au reste C se trouvent à l'état oxydé.

6. Peptide suivant la revendication 5, caractérisé en ce qu'il présente la séquence d'aminoacides qui suit:

7. Peptide suivant la revendication 5, caractérisé en ce qu'il présente la séquence d'aminoacides :

8. Peptide suivant l'une quelconque des revendications 1 à 4, caractérisé en ce qu'il présente la séquence d'aminoacides suivante :

9. Peptide suivant l'une quelconque des revendications précédentes, caractérisé en ce qu'il a été préparé par voie synthétique.

10. Peptide suivant l'une quelconque des revendications 1 à 9, caractérisé en ce qu'il a été préparé par recombinaison.

11. Fragment d'ADN isolé, caractérisé en ce qu'il code pour un peptide suivant l'une quelconque des revendications 1 à 4 ou 6 à 8.

12. Trousse d'essai pour la détermination d'anticorps contre des virus provoquant l'immunodéficience, caractérisée en ce que l'on met en oeuvre au moins un peptide suivant l'une quelconque des revendications 1 à 10.

13. Trousse d'essai suivant la revendication 12, caractérisée en ce qu'elle est un Western Blot ou immunotransfert.

14. Trousse d'essai suivant la revendication 13, caractérisée en ce qu'elle est un test ELISA ou un test de détermination d'anticorps par fluorescence.

15. Utilisation d'un peptide suivant l'une quelconque des revendications 1 à 10 pour la préparation de vaccins.
